# EUROPÄISCHE PATENTSCHRIFT

(11) **EP 3 993 768 B1**
(45) Veröffentlichungstag und Bekanntmachung des Hinweises auf die Patenterteilung: **31.12.2025**
(21) Anmeldenummer: 20730241.5
(22) Anmeldetag: 02.06.2020
(51) Int. Cl.: A61K 8/92, A61Q 19/00

(54) **LIPIDMISCHUNG AUS FLÜSSIGEN ÖLEN, HYDRIERTEN PFLANZENÖLEN UND SCHIBUTTER**
LIPID MIXTURE CONSISTING OF LIQUID OILS, HYDROGENATED VEGETABLE OILS, AND SHEA BUTTER
MÉLANGE LIPIDIQUE À BASE D'HUILES LIQUIDES, D'HUILES VÉGÉTALES HYDROGÉNÉES ET DE BEURRE DE KARITÉ

(30) Priorität: 02.07.2019 DE 102019209661
(43) Veröffentlichungstag der Anmeldung: 11.05.2022
(73) Patentinhaber: Beiersdorf AG, 22529 Hamburg (DE)
(72) Erfinder: KELLER, Julia, 21465 Wentorf (DE); NILSSON, Jan, 22177 Hamburg (DE)
(74) Vertreter: Beiersdorf AG
(86) Internationale Anmeldenummer: PCT/EP2020/065176
(87) Internationale Veröffentlichungsnummer: WO 2021/001104

(56) Entgegenhaltungen:
- DE-A1- 102005 003 708
- US-A1- 2004 198 620
- JUNE 2012: "Natural Oils", 1 June 2012 (2012-06-01), XP055721781, Retrieved from the Internet <URL:https://e-applications.basf-ag.de/data/basf-pcan/pds2/pds2-web.nsf/41578AEB351B7B87C125765700419624/$File/CEGESOFT_r__HF_52_E.pdf> [retrieved on 20200811]
- FERNANDE G. HONFO ET AL: "Nutritional Composition of Shea Products and Chemical Properties of Shea Butter: A Review", CRITICAL REVIEWS IN FOOD SCIENCE AND NUTRITION, vol. 54, no. 5, 21 January 2014 (2014-01-21), USA, pages 673 - 686, XP055721919, ISSN: 1040-8398, DOI: 10.1080/10408398.2011.604142

## Beschreibung

Die vorliegende Erfindung betrifft eine kosmetische Zubereitung enthaltend
a) 80-90 Gewichts-% unter Normalbedingungen flüssige Öle,
b) 8-12 Gewichts-% hydrierte Pflanzenöle (INCI: Hydrogenated Vegetable Oil),
c) 2-5 Gewichts-% Schibutter (INCI: Shea Butter),
wobei sich alle Gewichtsangaben auf das Gesamtgewicht der Zubereitung beziehen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Hautpflegeprodukte, in der Regel Crémes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Neben der Reinigung und Pflege der Haut haben Kosmetika auch eine ästhetische Aufgabe. Sie sollen das äußere Erscheinungsbild des Anwenders entsprechend den jeweiligen kulturellen Vorstellungen "verbessern". Kosmetika erfüllen damit eine psychologisch-soziale Funktion, da sie die (optische) Attraktivität der Anwender erhöhen. In diesen Bereich fällt vor allen Dingen die "dekorative" Kosmetik, die mit Hilfe von auf die Haut aufgetragenen Farbstoffen das Erscheinungsbild der Anwender verändert. Indirekt haben aber auch Reinigungs- und Pflegeprodukte einen positiven Einfluss, da eine saubere, gesunde Haut dem Schönheitsideal der Menschen entspricht.

Eine besondere Form kosmetischer Zubereitungen stellen Pflegeprodukte auf der Basis von Ölen und Wachsen dar. Diese haben gegenüber Emulsionen den Vorteil, im Wesentlichen wasserfrei zu sein, so dass auf den Einsatz von konservierenden Substanzen verzichtet werden kann. Da Konservierungsmittel beim Verbraucher unbeliebt sind, gibt es ein wachsendes Interesse an so genannten "konservierungsmittelfreien" Kosmetika.

Ein Nachteil des Standes der Technik besteht nun in dem Umstand, dass die herkömmlichen Pflegeöle in der Regel zu dünnflüssig (niedrigviskos) sind und sich daher relativ schlecht auf die Haut auftragen lassen. Oft kommt es bei dem Auftragungsprozess zu einem unkontrollierten "Heruntertropfen" der Zubereitung von der Haut und der ungewollten Entstehung von Fettflecken auf mit der Zubereitung kontaminierten Kleidungsstücken. Weicht der Fachmann zur Umgehung dieser Probleme auf wachsartige Zubereitungen aus, muss er nach dem Stande der Technik mit dem Nachteil leben, dass diese Zubereitungen in der Regel so zäh und fest (d.h. hochviskos) sind, dass sie sich nur schwer aus dem Vorratsbehältnis entnehmen und auf der Haut verteilen lassen.

Es war daher die Aufgabe der vorliegenden Erfindung, eine Lipid-basierte kosmetische Zubereitung zu entwickeln, die einerseits so fest ist, dass sie sich gezielt und kontrolliert auf der Haut auftragen lässt und andererseits so dünnflüssig ist, dass sie sich problemlos auf der Haut verteilen lässt.

Ein weiterer Nachteil von herkömmlichen, auf Lipiden basierenden Zubereitungen ist deren thermische Instabilität. Viele Hautöle und -wachse, die ja in der Regel Substanzmischungen unterschiedlicher lipider Verbindungen und Füllstoffe darstellen, neigen bei höheren Lagerungs- und Anwendungstemperaturen zu Instabilitäten, d.h. zum "Ausölen" und Ausfällungen von Inhaltsstoffen. Die Zubereitung verliert ihre Homogenität. Dieser in der Regel irreversible Prozess tritt meist schon bei Temperaturen von 40 °C auf, was insbesondere für die Lagerung während der Sommermonate oder in wärmeren Klimazonen ein Problem darstellt.

Es war daher die Aufgabe der vorliegenden Erfindung, eine thermisch stabile kosmetische Zubereitung auf Lipidbasis zu entwickeln.

Überraschend gelöst werden die Aufgaben durch eine kosmetische Zubereitung enthaltend
a) 80-90 Gewichts-% unter Normalbedingungen flüssige Öle,
b) 8-12 Gewichts-% hydrierte Pflanzenöle (INCI: Hydrogenated Vegetable Oil),
c) 2-5 Gewichts-% Schibutter (INCI: Shea Butter),
wobei sich alle Gewichtsangaben auf das Gesamtgewicht der Zubereitung beziehen.

Dabei werden erfindungsgemäß unter Normalbedingungen 20°C und 1,013 bar verstanden. Zwar kennt der Stand der Technik die US 2004/198620, die DE 10 2005 003708, die Publikation der BASF "Natural Oils, June 2012" (XP055721919) sowie die Veröffentlichung von Fernande G. Honfo et al. "Nutritional Composition of Shea Products and chemical Properties of Shea Butter: A Review" aus Critical Reviews in Food Science ans Nutrition, 54, 5, Seiten 673-686, ISSN 1040-8398, doch konnten diese Schriften nicht den Weg zur vorliegenden Erfindung weisen.

Die erfindungsgemäßen Zubereitungen zeigen zunächst eine salbenförmige/cremeförmige Konsistenz, die beim Verreiben auf der Haut dann überraschend in einen ölartigen Zustand übergeht. Diese durch das Verreiben Öl-artig gewordene Zubereitung zieht dann relativ schnell und rückstandsfrei in die Haut ein. Ein derartiges Verhalten bei der Applikation ist dem Fachmann bei Lipid-basierten Zubereitungen bisher unbekannt gewesen.

Die erfindungsgemäß vorteilhaften Ausführungsformen der vorliegenden Erfindung sind dadurch gekennzeichnet, dass als unter Normalbedingungen flüssige Öle ein oder mehrere Öle aus der Gruppe der Verbindungen Sonnenblumenöl (INCI: Helianthus Annuus Seed Oil), Mandelöl (Prunus amygdalus dulcis (*SweetAlmond*) *Oil*)), Capryl/Caprinsäure Triglyceride (INCI: Caprylic/Capric Triglycerid), Mineralöl, Octyldodecanol, Capryl/caprinsäure Koskosfettalkohol-Ester (INCI: Coco-Caprylate/Caprate), Cetearylisononanoat (INCI: Cetearyl Isononanoate), Arganöl (INCI: Argania Spinosa Kernel Oil) und/oder Mariendistelöl (INCI: Silybum Marianum Seed Oil) eingesetzt werden.

Die erfindungsgemäß bevorzugten Ausführungsformen der vorliegenden Erfindung sind dadurch gekennzeichnet, dass als unter Normalbedingungen flüssiges Öl Sonnenblumenöl (INCI: Helianthus Annuus Seed Oil) oder eine Mischung aus Octyldodecanol, Capryl/caprinsäure Koskosfettalkohol-Ester (INCI: Coco Caprylate/Caprate), Cetearylisononanoat (INCI: Cetearyl Isononanoate), Arganöl (INCI: Argania Spinosa Kernel Oil) und/oder Mariendistelöl (INCI: Silybum Marianum Seed Oil) eingesetzt wird.

Wird eine Mischung aus Octyldodecanol, Capryl/caprinsäure Koskosfettalkohol-Ester (INCI: Coco Caprylate/Caprate), Cetearylisononanoat (INCI: Cetearyl Isononanoate), Arganöl (INCI: Argania Spinosa Kernel Oil) und/oder Mariendistelöl (INCI: Silybum Marianum Seed Oil) eingesetzt, so ist das folgende Mischungsverhältnis besonders bevorzugt:
43 Gewichts-% Octyldodecanol
23 Gewichts-% Coco-Caprylate/Caprate
20 Gewichts-% Cetearyl Isononanoate
2 Gewichts-% Aganöl (Argania Spinosa Kernel Oil)
2 Gewichts-% Mariendistelöl (Silybum Marianum Seed Oil)

Es ist erfindungsgemäß vorteilhaft, wenn als hydrierte Pflanzenöle (INCI: Hydrogenated Vegetable Oil) eine Mischung aus Palmöl und Rapsöl eingesetzt wird.

Es ist insbesondere erfindungsgemäß von Vorteil, wenn das hydrierte Pflanzenöl einen Schmelzbereich von 50-57 °C aufweist. Dieses kann beispielsweise bei der BASF unter dem Handelsnamen Cegesoft HF 52 erworben werden.

Zwar kennt der Fachmann auch hydrierte Pflanzenöle (INCI: Hydrogenated Vegetable Oil) mit anderen Schmelzbereichen, beispielsweise Alkogel (Schmelzpunkt: 39°C), Kahlwax 6240 (Schmelzpunkt: 37-44 °C), Cutina HR Pulver (Schmelzpunkt 85-88 °C) oder Akofine P (Schmelzpunkt: 59-61 °C). Diese sind jedoch weit weniger gut geeignet.

Ferner sind die erfindungsgemäß vorteilhaften Ausführungsformen dadurch gekennzeichnet, dass das hydrierte Pflanzenöl einen Erstarrungsbereich von 40-53 °C aufweist.

Auch ist es vorteilhaft im Sinne der vorliegenden Erfindung, wenn das hydrierte Pflanzenöl bei 60 °C eine Dichte von 0,87-0,92 g/cm³ aufweist.

Es ist ferner erfindungsgemäß von Vorteil, wenn das hydrierte Pflanzenöl Triglyceride von C16-C18 gesättigten Fettsäuren und C18 ungesättigten Fettsäuren enthält.

Erfindungsgemäß bevorzugte Ausführungsformen der vorliegenden Erfindung sind dadurch gekennzeichnet, dass das hydrierte Pflanzenöl Triglyceride mit den Fettsäuren Palmitinsäure, Stearinsäure, Ölsäure, Eicosansäure und Behensäure enthält.

Vom erfindungsgemäß besonders bevorzugtem Cegesoft HF 52^{®} ist beispielsweise das folgende Mischungsverhältnis der einzelnen Fettsäuren offenbart:
38 % Palmitinsäure,
15 % Stearinsäure,
32 % Ölsäure,
3 % Eicosansäure und
11 % Behensäure.

Erfindungsgemäß bevorzugt beträgt das Gewichtsverhältnis von a) unter Normalbedingungen flüssige Öle zu b) hydrierten Pflanzenölen 10:1 bis 8:1 beträgt, wobei ein Gewichtsverhältnis von 9:1 besonders bevorzugt ist.

Die erfindungsgemäßen Zubereitungen werden darüber hinaus durch ihre Viskosität gekennzeichnet. So ist es erfindungsgemäß vorteilhaft, wenn die kosmetische Zubereitung eine Viskosität zwischen 3000 und 21000 mPas (gemessen mit dem Rheomat R123 der Firma proRheo, Spindel Nr. 1 bei 25 °C) aufweist.

Für die erfindungsgemäße Schibutter ist es vorteilhaft im Sinne der vorliegenden Erfindung, wenn die Schibutter zu mindestens 85 Gewichts-% aus Triglyceriden von C-16 bis C18 gesättigten Fettsäuren besteht.

Die erfindungsgemäßen Zubereitungen können darüber hinaus weitere kosmetische Inhaltsstoffe enthalten.

So ist es erfindungsgemäß von Vorteil, wenn die Zubereitung Füllstoffe, insbesondere Tapiokastärke enthält. Die erfindungsgemäß vorteilhafte Einsatzkonzentration für Tapiokastärke beträgt dabei 0,05 bis 1,0 Gewichts-%, bezogen auf das Gesamtgewicht der Zubereitung. Tapiokastärke hat dabei gegenüber anderen Füllstoffen wie beispielsweise Zinkoxid den Vorteil, dass die Zubereitung nicht zu fest wird.

Die kosmetische Zubereitung ist nach einem der vorhergehenden Ansprüche dadurch gekennzeichnet, dass die Zubereitung Antioxidantien, insbesondere Tocopherol und/oder Tocopherylacetat enthält. Diese können erfindungsgemäß vorteilhaft in einer Menge von 0,1 bis 1 Gewichts-% in der Zubereitung enthalten sein.

Darüber hinaus kann die erfindungsgemäße Zubereitung noch weitere kosmetische Inhaltsstoffe enthalten, beispielsweise Parfümstoffe oder Emulgatoren wie insbesondere Glyceryl Stearat Citrate, Sodium Stearoyl Glutamate, Glyceryl Stearate, Sodium Cetearyl Sulfate. Diese können bis zu einer Einsatzrate von 2,5 Gewichts-%, bevorzugt bis zu 0,5 Gewichts-%, besonders bevorzugt 0,1 Gewichts-%, jeweils bezogen auf das Gesamtgewicht der Zubereitung, eingesetzt werden.

Auch ist es möglich und erfindungsgemäß vorteilhaft, der erfindungsgemäßen Zubereitung Ester aus Fettalkoholen und Fettsäuren, beispielsweise Myristylmyristat zuzusetzen.

Ferner ist es möglich und erfindungsgemäß vorteilhaft, der erfindungsgemäßen Zubereitung Alkylether wie Ethylhexylglycerin zuzusetzen. Erfindungsgemäß vorteilhaft sind dabei Einsatzkonzentrationen von bis zu 1 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Dihydroxyaceton, 8-Hexadecen-1,16-dicarbonsäure, Glycerylglycose, (2-Hydroxyethyl)harnstoff, Hyaluronsäure und/oder deren Salze und/oder Licochalcon A enthält.

Erfindungsgemäß vorteilhaft sind dabei Einsatzkonzentrationen von bis zu 2 Gewichts-%, bezogen auf das Gesamtgewicht der Zubereitung.

Es ist erfindungsgemäß vorteilhaft, wenn die Zubereitung ein oder mehrere Alkandiole aus der Gruppe der Verbindungen Propylenglycol, Butylenglycol, 1,2-Pentandiol, 1,2-Hexandiol, 1,2-Octandiol, 1,2-Decandiol, 2-Methyl-1,3-propandiol enthält. Erfindungsgemäß vorteilhaft sind dabei Einsatzkonzentrationen von bis zu 1 Gewichts-%, bezogen auf das Gesamtgewicht der Zubereitung.

Darüber hinaus kann die erfindungsgemäße Zubereitung bis zu 3 Gewichts-%, bezogen auf das Gesamtgewicht der Zubereitung, an Pigmenten enthalten, wobei ein Gehalt von bis zu 1,5 Gewichts-%, bezogen auf das Gesamtgewicht, erfindungsgemäß bevorzugt ist.

Auch kann die erfindungsgemäße Zubereitung UV-Lichtschutzfilter enthalten.

Nicht zuletzt ist ein Wassergehalt von bis zu 5 Gewichts-%, bezogen auf Gesamtgewicht der Zubereitung möglich.

Erfindungsgemäß vorteilhafte Ausführungsformen der vorliegenden Erfindung sind dadurch gekennzeichnet dass die Zubereitung frei ist von Parabenen, Methylisothiazolinon, Chlormethylisothiazolinon und DMDM-Hydantoin.

Grundsätzlich ist es erfindungsgemäß von Vorteil, wenn die kosmetische Zubereitung frei ist von Mineralöl Mineralwachs, Silikonöl, und Silikonwachs.

Außerdem ist es erfindungsgemäß vorteilhaft, wenn die erfindungsgemäße Zubereitung frei ist von Polyethylenglycolethern und -estern und Polyestern.

Die erfindungsgemäßen Zubereitungen werden insbesondere erfindungsgemäß vorteilhaft verwendet als Massage- und Pflegeöl für Babys, zur Förderung der Hautelastizität für Schwangere, zur Bartpflege für Männer und zur Haarpflege

Erfindungsgemäß ist auch der Herstellungsverfahren der erfindungsgemäßen Zubereitung. Dabei lassen sich erfindungsgemäß zwei Verfahren unterscheiden:
Enthält die Zubereitung keinerlei Füllstoffe, so werden sämtliche Komponenten aufgeschmolzen und vermischt und anschließend unter konstantem Rühren auf Raumtemperatur wieder abgekühlt.

Enthält die Zubereitung hingegen Füllstoffe, so werden diese zu den aufgeschmolzenen und vermischten Inhaltsstoffen zugegeben und anschließend homogenisiert. Diese Homogenisierung erfolgt erfindungsgemäß vorteilhaft mittels Homocenta oder Ultraturrax. Nach dieser ersten Homogenisierung wird die Zubereitung auf Raumtemperatur abgekühlt und anschließend bei Raumtemperatur ein weiteres Mal homogenisiert.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| 1. |
|---|
| 10 Gew.-% Cegesoft HF 52 (Hydrogenated Vegetable Oil) |
| 1 Gew.-% Mandelöl (Prunus Amygdalus Dulcis Oil) |
| 86,95 Gew.-% Sonnenblumenöl (Helianthus Annuus Seed Oil) |
| 2 Gew.-% Schibutter (Butyrospermum Parkii Butter) |
| 0,05 Gew.-% Tocopherol |

| 2. |
|---|
| 10 Gew.-% Cegesoft HF 52 (Hydrogenated Vegetable Oil) |
| 86,84 Gew.-% Sonnenblumenöl (Helianthus Annuus Seed Oil) |
| 0,5 Gew.-% Mandelöl (Prunus Amygdalus Dulcis Oil) |
| 2 Gew.-% Schibutter (Butyrospermum Parkii Butter) |
| 0,26 Gew.-% Parfum |
| 0,05 Gew.-% Tocopherol |
| 0,35 Gew.-% Caprylyl Glycol |

| 3. |
|---|
| 10 Gew:-% Cegesoft HF 52 (Hydrogenated Vegetable Oil) |
| 86,69 Gew.-% Sonnenblumenöl (Helianthus Annuus Seed Oil) |
| 0,5 Gew.-% Mandelöl (Prunus Amygdalus Dulcis Oil) |
| 2 Gew.-% Schibutter (Butyrospermum Parkii Butter) |
| 0,26 Gew.-% Parfum |
| 0,05 Gew.-% Tocopherol |
| 0,5 Gew.-% Butylene Glycol |

## Patentansprüche

1. Kosmetische Zubereitung enthaltend
a) 80-90 Gewichts-% unter Normalbedingungen flüssige Öle,
b) 8-12 Gewichts-% hydrierte Pflanzenöle (INCI: Hydrogenated Vegetable Oil),
c) 2-5 Gewichts-% Schibutter (INCI: Shea Butter),
wobei sich alle Gewichtsangaben auf das Gesamtgewicht der Zubereitung beziehen.

2. Kosmetische Zubereitung nach Anspruch 1, **dadurch gekennzeichnet, dass** als unter Normalbedingungen flüssige Öle ein oder mehrere Öle aus der Gruppe der Verbindungen Sonnenblumenöl (INCI: Helianthus Annuus Seed Oil), Mandelöl (Prunus amygdalus dulcis (*SweetAlmond*) *Oil*)), Capryl/Caprinsäure Triglyceride (INCI: Caprylic/Capric Triglycerid, Mineralöl, Octyldodecanol, Capryl/caprinsäure Koskosfettalkohol-Ester (INCI: Coco-Caprylate/Caprate), Cetearylisononanoat (INCI: Cetearyl Isononanoate), Arganöl (INCI: Argania Spinosa Kernel Oil) und/oder Mariendistelöl (INCI: Silybum Marianum Seed Oil) eingesetzt werden.

3. Kosmetische Zubereitung nach einem der vorhergehenden Ansprüche, **dadurch gekennzeichnet, dass** als unter Normalbedingungen Flüssiges Öl Sonnenblumenöl (INCI: Helianthus Annuus Seed Oil) oder eine Mischung aus Octyldodecanol, Capryl/caprinsäure Koskosfettalkohol-Ester (INCI: Coco Caprylate/Caprate), Cetearylisononanoat (INCI: Cetearyl Isononanoate), Arganöl (INCI: Argania Spinosa Kernel Oil) und/oder Mariendistelöl (INCI: Silybum Marianum Seed Oil) eingesetzt wird.

4. Kosmetische Zubereitung nach einem der vorhergehenden Ansprüche, **dadurch gekennzeichnet, dass** als hydrierte Pflanzenöle (INCI: Hydrogenated Vegetable Oil) eine Mischung aus Palmöl und Rapsöl eingesetzt wird.

5. Kosmetische Zubereitung nach einem der vorhergehenden Ansprüche, **dadurch gekennzeichnet, dass** das hydrierte Pflanzenöl einen Schmelzbereich von 50-57 °C aufweist.

6. Kosmetische Zubereitung nach einem der vorhergehenden Ansprüche, **dadurch gekennzeichnet, dass** das hydrierte Pflanzenöl einen Erstarrungsbereich von 40-53 °C aufweist.

7. Kosmetische Zubereitung nach einem der vorhergehenden Ansprüche, **dadurch gekennzeichnet, dass** das hydrierte Pflanzenöl bei 60 °C eine Dichte von 0,87-0,92 g/cm³ aufweist.

8. Kosmetische Zubereitung nach einem der vorhergehenden Ansprüche, **dadurch gekennzeichnet, dass** das hydrierte Pflanzenöleine Triglyceride von C16-C18 gesättigten Fettsäuren und C18 ungesättigten Fettsäuren enthält.

9. Kosmetische Zubereitung nach einem der vorhergehenden Ansprüche, **dadurch gekennzeichnet, dass** das hydrierte Pflanzenöl Triglyceride mit den Fettsäuren Palmitinsäure, Stearinsäure, Ölsäure, Eicosansäure und Behensäure enthält.

10. Kosmetische Zubereitung nach einem der vorhergehenden Ansprüche, **dadurch gekennzeichnet, dass** die kosmetische Zubereitung eine Viskosität zwischen 3000 und 21000 mPas (gemessen mit dem Rheomat R123 der Firma proRheo, Spindel Nr. 1 bei 25 °C) aufweist.

11. Kosmetische Zubereitung nach einem der vorhergehenden Ansprüche, **dadurch gekennzeichnet, dass** die Schibutter zu mindestens 85 Gewichts-% aus Triglyceriden von C-16 bis C18 gesättigten Fettsäuren besteht.

12. Kosmetische Zubereitung nach einem der vorhergehenden Ansprüche, **dadurch gekennzeichnet, dass** das Gewichtsverhältnis von a) unter Normalbedingungen flüssige Öle zu b) hydrierten Pflanzenölen 10:1 bis 8:1 beträgt.

13. Kosmetische Zubereitung nach einem der vorhergehenden Ansprüche, **dadurch gekennzeichnet, dass** die Zubereitung Füllstoffe, insbesondere Tapiokastärke enthält.

14. Kosmetische Zubereitung nach einem der vorhergehenden Ansprüche, **dadurch gekennzeichnet, dass** die Zubereitung Antioxidantien, insbesondere Tocopherol und/oder Tocopherylacetat enthält.

## Claims

1. Cosmetic preparation comprising
a) 80-90% by weight of oils that are liquid under standard conditions,
b) 8-12% by weight of hydrogenated vegetable oils (INCI: Hydrogenated Vegetable Oil),
c) 2-5% by weight of shea butter (INCI: Shea Butter),
where all weight figures are based on the total weight of the preparation.

2. Cosmetic preparation according to Claim 1, **characterized in that** one or more oils from the group of compounds of sunflower oil (INCI: Helianthus Annuus Seed Oil), almond oil (Prunus amygdalus dulcis (*Sweet Almond*) Oil), caprylic/ capric triglyceride (INCI: Caprylic/Capric Triglyceride), mineral oil, octyldodecanol, caprylic/capric acid coconut fatty alcohol ester (INCI: Coco-Caprylate/Caprate), cetearyl isononanoate (INCI: Cetearyl Isononanoate), argan oil (INCI: Argania Spinosa Kernel Oil) and/or milk thistle oil (INCI: Silybum Marianum Seed Oil) are used as oils that are liquid under standard conditions.

3. Cosmetic preparation according to either of the preceding claims, **characterized in that** sunflower oil (INCI: Helianthus Annuus Seed Oil) or a mixture of octyldodecanol, caprylic/capric acid coconut fatty alcohol ester (INCI: Coco-Caprylate/Caprate), cetearyl isononanoate (INCI: Cetearyl Isononanoate), argan oil (INCI: Argania Spinosa Kernel Oil) and/or milk thistle oil (INCI: Silybum Marianum Seed Oil) is used as oil that is liquid under standard conditions.

4. Cosmetic preparation according to any of the preceding claims, **characterized in that** a mixture of palm oil and rapeseed oil is used as hydrogenated vegetable oils (INCI: Hydrogenated Vegetable Oil).

5. Cosmetic preparation according to any of the preceding claims, **characterized in that** the hydrogenated vegetable oil has a melting range of 50-57°C.

6. Cosmetic preparation according to any of the preceding claims, **characterized in that** the hydrogenated vegetable oil has a solidification range of 40-53°C.

7. Cosmetic preparation according to any of the preceding claims, **characterized in that** the hydrogenated vegetable oil has a density of 0.87-0.92 g/cm³ at 60°C.

8. Cosmetic preparation according to any of the preceding claims, **characterized in that** the hydrogenated vegetable oil comprises a triglycerides of C16-C18 saturated fatty acids and C18 unsaturated fatty acids.

9. Cosmetic preparation according to any of the preceding claims, **characterized in that** the hydrogenated vegetable oil comprises triglycerides with the fatty acids palmitic acid, stearic acid, oleic acid, eicosanoic acid and behenic acid.

10. Cosmetic preparation according to any of the preceding claims, **characterized in that** the cosmetic preparation has a viscosity between 3000 and 21 000 mPas (measured with the Rheomat R123 from proRheo, spindle no. 1 at 25°C).

11. Cosmetic preparation according to any of the preceding claims, **characterized in that** the shea butter consists of triglycerides of C16 to C18 saturated fatty acids to an extent of at least 85% by weight.

12. Cosmetic preparation according to any of the preceding claims, **characterized in that** the weight ratio of a) oils that are liquid under standard conditions to b) hydrogenated vegetable oils is 10:1 to 8:1.

13. Cosmetic preparation according to any of the preceding claims, **characterized in that** the preparation comprises fillers, in particular tapioca starch.

14. Cosmetic preparation according to any of the preceding claims, **characterized in that** the preparation comprises antioxidants, in particular tocopherol and/or tocopheryl acetate.

## Revendications

1. Préparation cosmétique contenant
a) 80-90% en poids d'huiles liquides dans des conditions normales,
b) 8-12% en poids d'huiles végétales hydrogénées (INCI : Hydrogenated Vegetable Oil),
c) 2-5% en poids de beurre de karité (INCI : Shea Butter),
toutes les indications en poids se rapportant au poids total de la préparation.

2. Préparation cosmétique selon la revendication 1, **caractérisée en ce qu'**on utilise, en tant qu'huiles liquides dans des conditions normales, une ou plusieurs huiles du groupe de composés huile de tournesol (INCI : Helianthus Annuus Seed Oil), huile d'amande (Prunus amygdalus dulcis (Sweet Almond) Oil)), triglycérides d'acide caprylique/caprique (INCI : Caprylic/Capric Triglycerid), huile minérale, octyldodécanol, ester d'alcool gras de coco de l'acide caprylique/caprique (INCI : Coco-Caprylate/Caprate), isononanoate de cétéaryle (INCI : Cetearyl Isononanoate), huile d'argan (INCI : Argania Spinosa Kemel Oil) et/ou huile de Chardon-Marie (INCI : Silybum Marianum Seed Oil).

3. Préparation cosmétique selon l'une des revendications précédentes, **caractérisée en ce qu'**on utilise, en tant qu'huile liquide dans des conditions normales, l'huile de tournesol (INCI : Helianthus Annuus Seed Oil) ou un mélange d'octyldodécanol, d'ester d'alcool gras de coco de l'acide caprylique/caprique (INCI : Coco Caprylate/Caprate), l'isononanoate de cétéaryle (INCI : Cetearyl Isononanoate), l'huile d'argan (INCI : Argania Spinosa Kemel Oil) et/ou l'huile de Chardon-Marie (INCI : Silybum Marianum Seed Oil).

4. Préparation cosmétique selon l'une des revendications précédentes, **caractérisée en ce qu'**on utilise, en tant qu'huiles végétales hydrogénées (INCI : Hydrogenated Vegetable Oil), un mélange d'huile de palme et d'huile de colza.

5. Préparation cosmétique selon l'une des revendications précédentes, **caractérisée en ce que** l'huile végétale hydrogénée présente une plage de fusion de 50-57°C.

6. Préparation cosmétique selon l'une des revendications précédentes, **caractérisée en ce que** l'huile végétale hydrogénée présente une plage de solidification de 40-53 °C.

7. Préparation cosmétique selon l'une des revendications précédentes, **caractérisée en ce que** l'huile végétale hydrogénée présente, à 60°C, une masse volumique de 0,87-0,92 g/cm³.

8. Préparation cosmétique selon l'une des revendications précédentes, **caractérisée en ce que** l'huile végétale hydrogénée contient un triglycérides d'acides gras saturés en C16-C18 et d'acides gras insaturés en C18.

9. Préparation cosmétique selon l'une des revendications précédentes, **caractérisée en ce que** l'huile végétale hydrogénée contient des triglycérides avec les acides gras acide palmitique, acide stéarique, acide oléique, acide eicosanoïque et acide béhénique.

10. Préparation cosmétique selon l'une des revendications précédentes, **caractérisée en ce que** la préparation cosmétique présente une viscosité entre 3000 et 21.000 mPa.s (mesurée à l'aide d'un Rheomat R123 de la société proRheo, axe n° 1 à 25°C).

11. Préparation cosmétique selon l'une des revendications précédentes, **caractérisée en ce que** le beurre de karité est constitué, à raison d'au moins 85% en poids, de triglycérides d'acides gras saturés en C-16 à C18.

12. Préparation cosmétique selon l'une des revendications précédentes, **caractérisée en ce que** le rapport pondéral de a) huiles liquides dans des conditions normales à b) huiles végétales hydrogénées représente 10:1 à 8:1.

13. Préparation cosmétique selon l'une des revendications précédentes, **caractérisée en ce que** la préparation contient des charges, en particulier de l'amidon de tapioca.

14. Préparation cosmétique selon l'une des revendications précédentes, **caractérisée en ce que** la préparation contient des antioxydants, en particulier du tocophérol et/ou de l'acétate de tocophéryle.
